(19) Europäisches Patentamt / European Patent Office / Office européen des brevets

(11) **EP 4 768 494 A1**

(12) **EUROPEAN PATENT APPLICATION**
published in accordance with Art. 153(4) EPC

(43) Date of publication:
01.07.2026 Bulletin 2026/27

(21) Application number: 24855323.2

(22) Date of filing: 19.04.2024

(51) International Patent Classification (IPC):
$C07J \ 9/00 \ ^{(2006.01)}$  $C07J \ 75/00 \ ^{(2006.01)}$
$B01D \ 11/04 \ ^{(2006.01)}$  $B01D \ 3/40 \ ^{(2006.01)}$

(52) Cooperative Patent Classification (CPC):
Y02P 20/54

(86) International application number:
PCT/CN2024/088970

(87) International publication number:
WO 2025/039592 (27.02.2025 Gazette 2025/09)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 22.08.2023 CN 202311060951

(71) Applicants:
• Zhejiang University
Hangzhou, Zhejiang 310058 (CN)
• Institute of Zhejiang University-Quzhou
Quzhou, Zhejiang 324000 (CN)

(72) Inventors:
• BAO, Zongbi
Hangzhou, Zhejiang 310058 (CN)

• DING, Zexiang
Hangzhou, Zhejiang 310058 (CN)
• CAO, Yifeng
Quzhou, Zhejiang 324000 (CN)
• CHEN, Lihang
Quzhou, Zhejiang 324000 (CN)
• YANG, Qiwei
Hangzhou, Zhejiang 310058 (CN)
• LIU, Baojian
Quzhou, Zhejiang 324000 (CN)
• ZHANG, Zhiguo
Hangzhou, Zhejiang 310058 (CN)
• REN, Qilong
Hangzhou, Zhejiang 310058 (CN)

(74) Representative: Biggi, Cristina
Bugnion S.p.A.
Viale Lancetti 17
20158 Milano (IT)

(54) **METHOD FOR SEPARATING CHOLIC ACID AND DEOXYCHOLIC ACID BY BIOCOMPATIBLE TWO-PHASE SYSTEM**

(57) Disclosed in the present invention is a method for extracting and separating cholic acid and deoxycholic acid by a liquid-liquid two-phase system and exhibiting good biocompatibility, low toxicity, and high efficiency. The method comprises: subjecting a feed solution containing an amino acid ionic liquid, and respectively obtaining high-purity cholic acid and deoxycholic acid from an extract liquid and a raffinate. Compared with conventional ionic liquids and organic solvent extractants, the amino acid ionic liquid extractant used in the present invention has the advantages of better degradability, higher biocompatibility, and higher separation efficiency, has the characteristics of large treatment capacity, simple process, low toxicity, environmental friendliness, and low costs, and is suitable for industrial production.

## Description

### Field of the Invention

The present invention belongs to the technical field of chemical separation, and specifically relates to a method for the extractive separation of cholic acid and deoxycholic acid using a highly biocompatible amino acid ionic liquid as a medium, which combines biocompatibility, low ionic liquid usage, and high processing capacity.

### Background of the Invention

Cholic acid and deoxycholic acid are steroid derivatives with various medical values. They have a broad application prospect. For example, they can be used to regulate the cholesterol metabolism in the body, dissolve lipids, treat the symptoms of cholecystitis and dyspepsia, and be applied to biochemical research, such as lysing cells and extracting nuclear proteins. No matter produced by separating from natural products or by chemical synthesis, cholic acid and deoxycholic acid often coexist as a mixture. Due to very similar molecular structures-the difference lies only in one hydroxyl group at the C7-position-making their efficient separation quite challenging.

Ionic liquids (ILs), composed entirely of anions and cations, are liquid at or near room temperature. Recently, ILs have attracted attention as a new class of "green" and efficient separation media in the field of separation. Compared with traditional organic solvent extractants, ILs exhibit unique advantages such as exceptional thermal/chemical stability, high cohesive energy that tends to form biphasic systems, enhanced solubility due to versatile interactions with solutes, and highly tunable structural properties. Satisfying separation efficiency for certain homologous with similar structure and properties has been achieved by designing the IL structures to adjust their physiochemical properties, and therefore intensity of interaction difference between ILs and solutes to be separated.

Although ionic liquids have long been considered as "green" solvents, an increasing number of research indicates that the potential toxicity of ionic liquids cannot be ignored. On the one hand, the acute toxicity of ionic liquids is closely related to their structure. On the other hand, the toxicity of ionic liquids is also reflected in the fact that they are not easily degraded in the environment, which can accumulate in the food chain, and affect the human body. Additionally, ionic liquids are more expensive than conventional solvents, so that the amount of ILs used in extraction process needs to be strictly controlled.

Cholic acid and deoxycholic acid are chemicals with medical value. It is necessary to consider the toxicity and cost of ionic liquids comprehensively when they are used as the separation media for cholic acid and deoxycholic acid, and use ionic liquids with good biocompatibility, large processing capacity, and high separation efficiency as far as possible to be applied to the separation of this system. It is evident that there is much room for improvement in the method of separating cholic acid and deoxycholic acid.

### Summary of the Invention

Given the deficiencies in the prior art, the present invention provides a method of separating cholic acid and deoxycholic acid from their mixture and obtaining high-purity products by multi-stage fractional extraction with an extractant containing an amino-acid-based ionic liquid. The amino-acid-based ionic liquids used in the present invention are more degradable and biocompatible than conventional ionic liquids. The present invention is featured with lower toxicity, lower amount of ionic liquid usage, and higher processing capacity, which are suitable for industrial production.

In order to achieve the above objects, the present invention provides a method of separating cholic acid and deoxycholic acid. The method comprises subjecting feed solution containing cholic acid and deoxycholic acid to fractional extraction using an amino-acid-based ionic liquid extractant.

In some embodiments, the ionic liquid consists of a cation and an amino acid anion.

In some embodiments, the cation is selected from at least one of the following cations, including an imidazolium cation, a quinolinium cation, a pyridinium cation, an isoquinolinium cation, a benzimidazolium cation, a quaternary ammonium cation, a quaternary phosphonium cation, a triazolium cation, a betainium cation, a guanidinium cation, a morphiazolium cation, a oxazolidinium cation, a cholinium cation, or a sulfonium salt cation; and the cation is optionally substituted with one or more substituents.

In some embodiments, the substituent is selected from at least one of amino, hydroxy, alkyl, alkenyl, phenyl, cyano group, thiocyano group, sulfonyl, or carbonyl groups. In some preferred embodiments, the alkyl group refers to a C1-C4 straight chain alkyl, the alkenyl group refers to a C1-C4 straight chain alkenyl, the cyano group refers to a C1-C4 straight chain cyano group, the thiocyano group refers to a C1-C4 straight chain thiocyano group, the sulfonyl group refers to a C1-C4 straight chain sulfonyl group, and the carbonyl refers to a C1-C4 straight chain carbonyl group. The number of substituents can be mono-substitution or multi-substitution, such as di-substitution or tri-substitution, and the substituents at different sites in the multi-substitution may be the same or different.

In some embodiments, the amino acid anion is selected from carboxylate anions containing amino, sec-amino and/or tert-amino in the molecule. In some embodiments, the amino acid anion is selected from at least one of a neutral amino acid anion, a basic amino acid anion, and an acidic amino acid anion. In some specific embodiments, the amino acid anion includes, but is not limited to, at least one of a neutral amino acid anion, such as a glycinate anion, a prolinate anion, an alaninate anion, a leucinate anion, a valinate anion, and a phenylalaninate anion; a basic amino acid anion, such as a tryptophanate anion, an asparaginate anion, a glutamate anion, a lysinate anion, and an argininate anion; sulfur-containing amino acid anion, such as cysteinate anion and methioninate anion; or an acidic amino acid anion, such as glutamate anion.

In some embodiments, the amino acid anion is optionally substituted by one or more substituents.

In some embodiments, the substituent is selected from at least one of amino, hydroxy, alkyl, alkenyl, phenyl, cyano group, thiocyano group, sulfonyl, or carbonyl. In some preferred embodiments, the alkyl refers to a C1-C4 straight chain alkyl, the alkenyl refers to a C1-C4 straight chain alkenyl, the cyano group refers to a C1-C4 straight chain cyano group, the thiocyano group refers to a C1-C4 straight chain thiocyano group, the sulfonyl refers to a C1-C4 straight chain sulfonyl group, and the carbonyl refers to a C1-C4 straight chain carbonyl group. The number of substituents can be mono-substitution or multi-substitution, such as di-substitution or tri-substitution, and the substituents at different sites in the multi-substitution may be the same or different.

In some specific embodiments, the ionic liquid is selected from at least one of 1-ethyl-3-methylimidazolium amino acid ionic liquid, choline amino acid ionic liquid, tert-butylammonium amino acid ionic liquid, 1-ethyl-1-methylpiperidinium phosphate amino acid ionic liquid, 1-ethyl-1-methylpyrrolidinium amino acid ionic liquid, 1-hydroxyethyl-3-methylimidazolium amino acid ionic liquid, betainium amino acid ionic liquid, and 1-butyl-2,3-dimethylimidazolium amino acid ionic liquid.

In some specific embodiments, the ionic liquid is selected from at least one of betainium alaninate ionic liquid, cholinium prolinate ionic liquid, cholinium serinate ionic liquid, or cholinium argininate ionic liquid.

Amino acids are the smallest unit of proteins constituting living organisms, most of which originate from are of biological origin and are diverse, inexpensive, and biodegradable, so amino acid ionic liquids have good biocompatibility. In addition, amino acid anions have a significant advantage of multiple intermolecular hydrogen-bonding interaction sites because they contain carboxylate anions and amino groups, and have a stronger ability to bind hydrogen atoms as hydrogen bonding acceptors and more sites of action than carboxylate anions. Cholic acid and deoxycholic acid molecules differ in their ability to form hydrogen bonds due to the difference in the number of hydroxyl groups; therefore, using amino-acid-based ionic liquids as separation medium could combine high selectivity with good biocompatibility in the separation of cholic acid and deoxycholic acid differing in the number of hydroxyl groups.

In some embodiments, the method further comprises concentrating and drying the raffinate of the fractional extraction to obtain deoxycholic acid. In some specific embodiments, the raffinate of the fractional extraction is vacuum concentrated, washed with water and dried to obtain deoxycholic acid.

In some embodiments, the method further comprises back-extracting the extract phase of the fractional extraction process, concentrating, and drying the back-extracted phase to obtain cholic acid. In some specific embodiments, the extract of the fractional extraction is back-extracted, and the back-extracted phase is vacuum concentrated, washed with water, and dried to obtain cholic acid.

In some embodiments, taking product quality and production cost into account, the flow ratio of the extractant, scrubbing solvent, and feed solution is 1: (0.5-5): (0.1-10), preferably 1: (0.75-1): (0.25-5) in the fractional extraction. In some specific embodiments, the flow ratio of the extractant, scrubbing solvent, and feed solution is 4:3:1 or 1:1:5.

In some embodiments, the mass fraction of ionic liquid in the extractant is 0.01%-100%, for example, 0.01%, 0.05%, 0.1%, 0.5%, 1%, 5%, 10%, 20%, 30%, 40%, 50%, 60%, 70%, 80%, 90%, 100%, or any value therebetween. In some preferred embodiments, the mass fraction of ionic liquid in the extractant is 0.01%-20%, more preferably 0.05%-5%, further preferably 0.05%-2%.

In some embodiments, the total concentration of cholic acid and deoxycholic acid in the feed solution is 0.1 g/L-100 g/L, e.g., 0.2 g/L, 0.5 g/L, 1 g/L, 10 g/L, 50 g/L, 100 g/L, or any value therebetween. The mixture of cholic acid and deoxycholic acid in the feed solution of the present invention has a total weight percentage of cholic acid and deoxycholic acid of 90% or more. The mixture of cholic acid and deoxycholic acid can be purchased directly from the market, or an animal bile acid extract with a low content of cholic acids can be refined by well-known steps of extraction, precipitation to obtain a feed in which the total mass percentage of cholic acid and deoxycholic acid is 90% or more.

In some embodiments, the feed solution further comprises a feed solvent, preferably the feed solvent is selected from C4-C8 straight or branched chain alcohol . In some preferred embodiments, the feed solvent is selected from at least one of n-butanol, 2-butanol, iso-butanol, n-pentanol, n-hexanol, 2-hexanol, n-heptanol, iso-octanol, and iso-amyl alcohol.

In some embodiments, the extractant further comprises a diluent to reduce viscosity and modulate extractant properties. In some embodiments, the diluent is selected from polar solvents and non-polar solvents. In some preferred embodiments, for hydrophilic ionic liquids, the diluent is selected from polar solvents such as water, dimethyl sulfoxide, and the like. In some preferred embodiments, for hydrophobic ionic liquids, the diluent is selected from a non-polar solvent such as hexane.

In some embodiments, the mass fraction of the diluent in the extractant is 0.01%-99.9%, for example, 0.01%, 0.1%, 1%, 5%, 10%, 20%, 30%, 40%, 50%, 60%, 70%, 80%, 82.5%, 85%, 87.5%, 90%, 92.5%, 95%, 97.5%, 98%, 99%, 99.2%, 99.4%, 99.6%, 99.8%, 100% or any value between them. In some preferred embodiments, the mass fraction of the diluent in the extractant is 0.01%-20%, more preferably 0.05%-5%, further preferably 0.05%-2%.

In some embodiments, the scrubbing solvent is selected from C4-C8 straight or branched chain alcohol compounds. In some preferred embodiments, the scrubbing solvent is selected from at least one of n-butanol, 2-butanol, iso-butanol, n-pentanol, n-hexanol, 2-hexanol, n-heptanol, iso-octanol, and iso-amyl alcohol. In a further preferred embodiment, the scrubbing solvent is the same as the feed solvent and can form a liquid-liquid biphasic system with less mutual solubility with the extractant while having a better solubility for cholic acids.

In some embodiments, the operational temperature of 20°C-70°C of the fractional extraction is 20°C-70°C, such as 20°C, 30°C, 40°C, 50°C, 60°C, 70°C, or any value therebetween. If the temperature is too low, the viscosity of the extractant is higher, the mass transfer rate is reduced, and the processing capacity is small, which is not conducive to production operations; if the temperature is too high, the solvent is heavily volatilized, the distribution ratio and selectivity of the fractional extraction will be reduced.

In some embodiments, the fractional extraction is a multi-stage fractional extraction.

In some embodiments, the fractional extraction employs a fractional extraction operation comprising an extraction section and a washing section. The extractant enters the fractional extraction system from the first stage of the extraction section. The feed solution enters from the last stage of the extraction section. The scrubbing solvent enters from the first stage of the washing section, and combines with the feed solution and enters the extraction section in the last stage of the extraction section. The extracting phase and the raffinate phase are in multi-stage counter-current contact The deoxycholic acid-enriched raffinate flows out of the first stage of the extraction section, which is then vacuum-concentrated, washed with water and dried to obtain deoxycholic acid. The cholic acid-enriched extract solution flows out from the first stage of the washing section, which is then subjected to back-extraction, vacuum concentrated, washed with water, and dried to obtain cholic acid. The absolute purity of the cholic acid obtained by the separation method of the present invention is > 90%; the relative content ratio of cholic acid to deoxycholic acid is > 95:5 (mass ratio, w:w).

In some embodiments, the method comprises the following steps:

(1) Feed solution, mixture of feed solvent, cholic acid and deoxycholic acid is prepared in fractional extraction. Binary solvent consisting of an amino acid ionic liquid and a diluent is used as an extractant. Solvent identical to the feed solvent is used as scrubbing solvent. The fractional extraction is divided into an extraction section and a washing section. The extractant enters the fractional extraction system from the first stage of the extraction section, the feed solution enters the fractional extraction system from the last stage of the extraction section, the scrubbing solvent enters the fractional extraction system from the first stage of the washing section. The feed solution is combined with the scrubbing solvent in the last stage of the extraction section and then enter the extraction section together. The extract phase and the raffinate phase are subjected to multi-stage counter-current extraction. The cholic acid-enriched extract flows out of the first stage of the washing section. The deoxycholic acid-enriched raffinate flows out of the first stage of the extraction section.

(2) The raffinate from the Step (1) is vacuum-concentrated, washed with water and dried to obtain deoxycholic acid; the extract from the step (1) is back-extracted with the same solvent as the feed solvent and scrubbing solvent, and the solvent-enriched phase is vacuum-concentrated, washed with water and dried to give cholic acid, and the ionic liquid-enriched phase is vacuum-concentrated for recycling.

The advantages of the present invention are as follows:

1. The present invention adopts an extractant containing amino-acid-based ionic liquid for fractional extraction. This method has good selective separation ability for cholic acid and deoxycholic acid whose molecular structure differs by only one hydroxyl group. The process is simple, with less energy consumption, safe, and environmentally friendly.
2. The amino-acid-based ionic liquid used in the present invention has good degradability and biocompatibility, reduces the impact on the human body and the environment, and has a broad and green application prospect.
3. The amino acid-based ionic liquids used in the present invention possess high processing capacity with low amount in extractant, which can reduce the viscosity of the extractant and the production cost, and have industrial application potential.

## Detailed Description of the Invention

The present invention provides a method for separating cholic acid and deoxycholic acid with low toxicity, good biocompatibility, high processing capacity, simple process and high efficiency, which employs fractional extraction using an extractant containing an amino acid-based ionic liquid in which amino acid as an anion. Amino acid-based ionic liquids have attracted attention as a new class of green separation media in the field of separation. Compared with conventional ionic liquids, amino acid-based ionic liquids as extractants have some unique properties, such as good biocompatibility, degradability, safety, and environmental protection; in addition, amino acid ionic liquids have amino and carboxylate anions, which can form more sites for intermolecular forces such as hydrogen bonding with solutes and have stronger effects. The anionic and cationic structure of the ionic liquids can be designed to regulate the interaction types and strengths between ionic liquids and the compounds to be separated, so as to achieve efficient molecular recognition and separation.

The molecular structures of cholic acid and deoxycholic acid are very similar, with the difference lying in the hydroxyl group at the C-7 position in cholic acid, which leads to different ability to form hydrogen bonding. According to the structural characteristics and differences between the structures of cholic acid and deoxycholic acid, the use of amino acid ionic liquids with strong hydrogen-bonding as the extractant can not only strengthen the strength and differences of the interaction between cholic acid and the extractant, improve the processing capacity and selectivity, but also reduce the amount of ionic liquids in the extractant and control the production cost. If the concentration of ionic liquid in the extractant is too large, it will lead to an increase in the amount of ionic liquid consumed to produce a unit of product quality, an increase in cost, as well as a high viscosity of the extractant, and a low mass transfer rate. The extractants containing amino-acid-based ionic liquids employed in the present invention are capable of efficient selective separation of cholic acid and deoxycholic acid with low-concentration ionic liquid as extractant.

For the mixture containing cholic acid and deoxycholic acid, the distribution coefficients of cholic acid and deoxycholic acid were only 0.0257 and 0.0117 when single-stage extraction was carried out with n-butanol as the solvent and pure water as the extractant at 30°C, which was not suitable for industrial production. The distribution coefficients of cholic acid and deoxycholic acid increases to 1.36 and 0.58 with the selectivity coefficient of cholic acid/deoxycholic acid up to 2.4, which is suitable for industrial production, when 1 wt.% choline glycinate aqueous solution is used as the extractant. It can be seen that, compared to the situation when pure water was used as extractant, the addition of only 1 wt.% choline glycinate significantly improved the distribution coefficients of cholic acid and deoxycholic acid while maintaining a high separation selectivity.

Based on the above findings, the present invention proposes a method of separating cholic acid and deoxycholic acid and obtaining high-purity components by multi-stage fractional extraction using a binary solvent consisting of an amino-acid-based ionic liquid and a diluent as an extractant.

The invention is further described with specific examples shown below. It should be noticed that these examples are intended only to illustrate the invention and not to limit the protection scope of the present invention.

In the following examples, the concentrations of cholic acid and deoxycholic acid were analyzed by high performance liquid chromatography (HPLC). The specific analytical conditions of HPLC were as follows: separation was achieved on a Waters Sunfire C18 column (4.6 mm×250 mm, particle size 5 $\mu$m) maintained at 30 °C, the mobile phase was composed of methanol: acetonitrile: 0.1% formic acid aqueous solution = 63:17:20 (v/v/v) at a flow rate of 0.8 mL/min. Due to the weak UV response of cholic acid and deoxycholic acid, a universal charged aerosol detector (CAD, Thermo Fisher) was used.

In the following examples, the yield and purity are calculated according to the following equations.

$$\text{Yield} = \text{mass of cholic acid in the product/mass of cholic acid in the feed} \times 100\%;$$

$$\text{Purity} = \text{mass of bile acid in the product/total mass of the product} \times 100\%.$$

**Example 1**

A mixture of feed cholic acid and deoxycholic acid (where the total mass percentage of cholic acid and deoxycholic acid is 99%) was dissolved in n-butanol to formulate a feed solution with a total concentration of 20 g/L. Cholinium prolinate ionic liquid-water binary solvent was used as extractant (the mass fraction of cholinium prolinate ionic liquid is 1%). n-butanol was used as scrubbing solvent. The flow ratio of the extractant, scrubbing solvent, and feed solution was 4:3:1 (v/v/v), and the fractional extraction was carried out at 30 °C. The fractional extraction was divided into an extraction section and a washing section (the extraction section of a total of 6 stages, the washing section of a total of 3 stages). The extractant enters the fractional extraction system from the first stage of the extraction section, the feed solution enters the fractional extraction system from the last stage of the extraction section, and the scrubbing solvent enters the fractional extraction system from the first stage of the washing section. The cholic acid-enriched extract flows out of the first stage of the washing section, and the deoxycholic acid-enriched raffinate flows out of the first stage of the extraction, which were collected, respectively.

The raffinate was vacuum-concentrated, washed with water and dried to obtain deoxycholic acid. The extract was diluted 10 times, subjected to back-extraction 3 times by 1/10 volume of n-butanol at 30°C The n-butanol phases were combined, vacuum concentrated, washed with water and dried to give cholic acid. In the product, the purity was 89.35% and the yield was 93.57% for cholic acid and 85.06% and 93.65% for deoxycholic acid. The ionic liquid-water phase after back-extraction is concentrated to increase the ionic liquid concentration, which can be recovered and recycled.

## Example 2

A mixture of feed cholic acid and deoxycholic acid (where the total mass percentage of cholic acid and deoxycholic acid is 99%) was dissolved in n-butanol to formulate a feed solution with a total concentration of 20 g/L. Betainium alaninate ionic liquid-water binary solvent was used as extractant (the mass fraction of betainium alaninate ionic liquid is 1%), n-butanol was used as scrubbing solvent. The flow ratio of the extractant, scrubbing solvent, and feed solution was 4:3:1 (v/v/v), and the fractional extraction was carried out at 30 °C. The fractional extraction was divided into an extraction section and a washing section (the extraction section of a total of 6 stages, the washing section of a total of 3 stages). The extractant enters the fractional extraction system from the first stage of the extraction section, the feed solution enters the fractional extraction system from the last stage of the extraction section, and the scrubbing solvent enters the fractional extraction system from the first stage of the washing section. The cholic acid-enriched extract flows out of the first stage of the washing section, and the deoxycholic acid-enriched raffinate flows out of the first stage of the extraction, which were collected, respectively.

The raffinate was vacuum-concentrated, washed with water and dried to obtain deoxycholic acid. The extract was diluted 10 times, subjected to back-extraction 3 times by 1/10 volume of n-butanol at 30°C. The n-butanol phases were combined, vacuum concentrated, washed with water and dried to give cholic acid. In the product, the purity was 91.36% and the yield was 91.31% for cholic acid and 91.31% and 91.37% for deoxycholic acid. The ionic liquid-water phase after back-extraction is concentrated to increase the ionic liquid concentration, which can be recovered and recycled.

## Example 3

A mixture of feed cholic acid and deoxycholic acid (where the total mass percentage of cholic acid and deoxycholic acid is 99%) was dissolved in n-heptanol to formulate a feed solution with a total concentration of 20 g/L. Betainium alaninate ionic liquid-dimethyl sulfoxide binary solvent was used as extractant (the mass fraction of betainium alaninate ionic liquid is 1%). n-heptanol was used as scrubbing solvent. The flow ratio of the extractant, scrubbing solvent, and feed solution was 4:3:1 (v/v/v) The fractional extraction was carried out at 30 °C, the fractional extraction was divided into an extraction section and a washing section (the extraction section of a total of 6 stages, the washing section of a total of 3 stages). The extractant enters the fractional extraction system from the first stage of the extraction section, the feed solution enters the fractional extraction system from the last stage of the extraction section, and the scrubbing solvent enters the fractional extraction system from the first stage of the washing section. The cholic acid-enriched extract flows out of the first stage of the washing section, and the deoxycholic acid-enriched raffinate flows out of the first stage of the extraction, which were collected, respectively.

The raffinate was vacuum-concentrated, washed with water and dried to obtain deoxycholic acid The extract was diluted 10 times, subjected to back-extraction 3 times by 1/10 volume of n-heptanol at 30°C. The n-heptanol phases were combined, vacuum concentrated, washed with water and dried to give cholic acid. In the product, the purity was 79.53% and the yield was 90.35% for cholic acid and 65.38% and 76.98% for deoxycholic acid. The ionic liquid- the diluent phase after back-extraction is concentrated to increase the ionic liquid concentration, which can be recovered and recycled.

## Example 4

A mixture of feed cholic acid and deoxycholic acid (where the total mass percentage of cholic acid and deoxycholic acid is 99%) was dissolved in n-butanol to formulate a feed solution with a total concentration of 20 g/L. Betainium alaninate ionic liquid-water binary solvent was used as extractant (the mass fraction of betainium alaninate ionic liquid is 0.08%). n-butanol was used as scrubbing solvent. The flow ratio of the extractant, scrubbing solvent, and feed solution was 4:3:1 (v/v/v). The fractional extraction was carried out at 30 °C. The fractional extraction was divided into an extraction section and a washing section (the extraction section of a total of 6 stages, the washing section of a total of 3 stages). The extractant enters the fractional extraction system from the first stage of the extraction section, the feed solution enters the fractional extraction system from the last stage of the extraction section, and the scrubbing solvent enters the fractional extraction system from the first stage of the washing section. The cholic acid-enriched extract flows out of the first stage of the washing section, and the deoxycholic acid-enriched raffinate flows out of the first stage of the extraction, which were collected, respectively.

The raffinate was vacuum-concentrated, washed with water and dried to obtain deoxycholic acid. The extract was diluted 10 times, subjected to back-extraction 3 times by 1/10 volume of n-butanol at 30°C. The n-butanol phases were combined, vacuum concentrated, washed with water and dried to give cholic acid. In the product, the purity was 85.69% and the yield was 93.10% for cholic acid and 87.94% and 92.65% for deoxycholic acid. The ionic liquid-water phase after back-extraction is concentrated to increase the ionic liquid concentration, which can be recovered and recycled.

**Example 5**

A mixture of feed cholic acid and deoxycholic acid (where the total mass percentage of cholic acid and deoxycholic acid is 99%) was dissolved in 2-heptanol to formulate a feed solution with a total concentration of 20 g/L. Cholinium serinate ionic liquid-water binary solvent was used as extractant (the mass fraction of cholinium serinate ionic liquid is 1%). 2-heptanol was used as scrubbing solvent. The flow ratio of the extractant, scrubbing solvent, and feed solution was 4:3:1 (v/v/v), and the fractional extraction was carried out at 30 °C. The fractional extraction was divided into an extraction section and a washing section (the extraction section of a total of 6 stages, the washing section of a total of 3 stages). The extractant enters the fractional extraction system from the first stage of the extraction section, the feed solution enters the fractional extraction system from the last stage of the extraction section, and the scrubbing solvent enters the fractional extraction system from the first stage of the washing section. The cholic acid-enriched extract flows out of the first stage of the washing section, and the deoxycholic acid-enriched raffinate flows out of the first stage of the extraction, which were collected, respectively.

The raffinate was vacuum-concentrated, washed with water and dried to obtain deoxycholic acid. The extract was diluted 10 times, subjected to back-extraction 3 times by 1/10 volume of 2-heptanol at 30°C. The 2-heptanol phases were combined, vacuum concentrated, washed with water and dried to give cholic acid. In the product, the purity was 87.21% and the yield was 93.87% for cholic acid and 86.48% and 90.79% for deoxycholic acid. The ionic liquid-enriched phase after back-extraction is concentrated to increase the ionic liquid concentration to the initial concentration for recycling.

**Example 6**

A mixture of feed cholic acid and deoxycholic acid (where the total mass percentage of cholic acid and deoxycholic acid is 99%) was dissolved in 2-heptanol to formulate a feed solution with a total concentration of 20 g/L. Cholinium serinate ionic liquid-water binary solvent was used as extractant (the mass fraction of cholinium serinate ionic liquid is 1%). 2-heptanol was used as scrubbing solvent. The flow ratio of the extractant, scrubbing solvent, and feed solution was 4:3:1 (v/v/v). The fractional extraction was carried out at 50 °C. The fractional extraction was divided into an extraction section and a washing section (the extraction section of a total of 6 stages, the washing section of a total of 3 stages). The extractant enters the fractional extraction system from the first stage of the extraction section, the feed solution enters the fractional extraction system from the last stage of the extraction section, and the scrubbing solvent enters the fractional extraction system from the first stage of the washing section. The cholic acid-enriched extract flows out of the first stage of the washing section, and the deoxycholic acid-enriched raffinate flows out of the first stage of the extraction, which were collected, respectively.

The raffinate was vacuum-concentrated, washed with water and dried to obtain deoxycholic acid. The extract was diluted 10 times, subjected to back-extraction 3 times by 1/10 volume of 2-heptanol at 30°C. The 2-heptanol phases were combined, vacuum concentrated, washed with water and dried to give cholic acid. In the product, the purity was 75.64% and the yield was 86.79% for cholic acid and 70.80% and 81.65% for deoxycholic acid. The ionic liquid-enriched phase after back-extraction is concentrated to increase the ionic liquid concentration to the initial concentration for recycling.

**Example 7**

A mixture of feed cholic acid and deoxycholic acid (where the total mass percentage of cholic acid and deoxycholic acid is 99%) was dissolved in n-butanol to formulate a feed solution with a total concentration of 20 g/L. Cholinium argininate ionic liquid-water binary solvent was used as extractant (the mass fraction of cholinium argininate ionic liquid is 1%). n-butanol was used as scrubbing solvent. The flow ratio of the extractant, scrubbing solvent, and feed solution was 1:1:5 (v/v/v). The fractional extraction was carried out at 30 °C. The fractional extraction was divided into an extraction section and a washing section (the extraction section of a total of 6 stages, the washing section of a total of 3 stages). The extractant enters the fractional extraction system from the first stage of the extraction section, the feed solution enters the fractional extraction system from the last stage of the extraction section, and the scrubbing solvent enters the fractional extraction system from the first stage of the washing section. The cholic acid-enriched extract flows out of the first stage of the washing section, and the deoxycholic acid-enriched raffinate flows out of the first stage of the extraction, which were collected, respectively.

The raffinate was vacuum-concentrated, washed with water and dried to obtain deoxycholic acid. The extract was diluted 10 times, subjected to back-extraction 3 times by 1/10 volume of n-butanol at 50°C. The n-butanol phases were combined, vacuum concentrated, washed with water and dried to give cholic acid. In the product, the purity was 97.32% and the yield was 60.37% for cholic acid and 71.42% and 93.58% for deoxycholic acid. The ionic liquid-enriched phase after back-extraction is concentrated to increase the ionic liquid concentration to the initial concentration for recycling.

**Comparative Example 1**

A mixture of feed cholic acid and deoxycholic acid (where the total mass percentage of cholic acid and deoxycholic acid is 99%) was dissolved in n-butanol to formulate a feed solution with a total concentration of 20 g/L. 1-ethyl-3-methylimidazolium chloride-water binary solvent was used as extractant (the mass fraction of 1-ethyl-3-methylimidazolium chloride is 1%), n-butanol was used as scrubbing solvent, the flow ratio of the extractant, scrubbing solvent, and feed solution was 4:3:1 (v/v/v). The fractional extraction was carried out at 30 °C. The fractional extraction was divided into an extraction section and a washing section (the extraction section of a total of 6 stages, the washing section of a total of 3 stages). The extractant enters the fractional extraction system from the first stage of the extraction section, the feed solution enters the fractional extraction system from the last stage of the extraction section, and the scrubbing solvent enters the fractional extraction system from the first stage of the washing section. The cholic acid-enriched extract flows out of the first stage of the washing section, and the deoxycholic acid-enriched raffinate flows out of the first stage of the extraction, which were collected, respectively.

The raffinate was vacuum-concentrated, washed with water and dried to give deoxycholic acid. The extract was diluted 10 times, subjected to back-extraction 3 times by 1/10 volume of n-butanol at 30°C. The n-butanol phases were combined, vacuum concentrated, washed with water and dried to give cholic acid. In the product, the purity was 66.58% and the yield was 4.21% for cholic acid and 43.97% and 96.48% for deoxycholic acid. The ionic liquid-enriched phase after back-extraction is concentrated to increase the ionic liquid concentration to the initial concentration for recycling.

**Comparative Example 2**

A mixture of feed cholic acid and deoxycholic acid (where the total mass percentage of cholic acid and deoxycholic acid is 99%) was dissolved in n-butanol to formulate a feed solution with a total concentration of 20 g/L. Tetramethylammonium chloride ionic liquid-water binary solvent was used as extractant (the mass fraction of tetramethylammonium chloride ionic liquid is 1%). n-butanol was used as scrubbing solvent. The flow ratio of the extractant, scrubbing solvent, and feed solution was 4:3:1 (v/v/v). The fractional extraction was carried out at 30 °C. The fractional extraction was divided into an extraction section and a washing section (the extraction section of a total of 6 stages, the washing section of a total of 3 stages). The extractant enters the fractional extraction system from the first stage of the extraction section, the feed solution enters the fractional extraction system from the last stage of the extraction section, and the scrubbing solvent enters the fractional extraction system from the first stage of the washing section. The cholic acid-enriched extract flows out of the first stage of the washing section, and the deoxycholic acid-enriched raffinate flows out of the first stage of the extraction, which were collected, respectively.

The raffinate was vacuum-concentrated, washed with water and dried to give deoxycholic acid. The extract was diluted 10 times, subjected to back-extraction 3 times by 1/10 volume of n-butanol at 30°C. The n-butanol phases were combined, vacuum concentrated, washed with water and dried to give cholic acid. In the product, the purity was 60.58% and the yield was 6.87% for cholic acid and 41.60% and 98.71% for deoxycholic acid. The ionic liquid-enriched phase after back-extraction is concentrated to increase the ionic liquid concentration to the initial concentration for recycling.

The above various examples are intended only to illustrate the technical solutions of the present application and are not intended to be a limitation thereof. Although the present application has been described in detail with reference to the foregoing examples, those skilled in the art should understand that it is still possible to modify the technical solutions recorded in the foregoing examples or to make equivalent alternative for some or all of the technical features therein. These modifications or alternatives do not take the essence of the corresponding technical solution out of the scope of the technical solution of this application.

**Claims**

1. A method of separating cholic acid and deoxycholic acid, the method comprising subjecting a feed solution containing cholic acid and deoxycholic acid to fractional extraction using an extractant containing an amino acid-based ionic liquid.

2. The method according to claim 1, wherein the ionic liquid consists of a cation and an amino acid anion,

wherein, the cation is selected from at least one of imidazolium cation, quinolinium cation, pyridinium cation, isoquinolinium cation, benzimidazolium cation, quaternary ammonium cation, quaternary phosphonium cation, triazolium cation, betainium cation, guanidinium cation, morphiazolium cation, oxazolidinium cation, cholinium cation, or sulfonium salt cation, and the cation is optionally substituted with one or more substituents;

the amino acid anion is selected from carboxylic acidate anions containing amino, sec-amino and/or tert-amino, preferably, the amino acid anion is selected from at least one of a neutral amino acid anion, a basic amino acid anion, and an acidic amino acid anion,

more preferably, the neutral amino acid anion comprises at least one of glycinate anion, prolinate anion, alaninate anion, leucinate anion, valinate anion, or phenylalaninate anion,

more preferably, the basic amino acid anion comprises at least one of tryptophanate anion, asparaginate anion, glutamate anion, lysinate anion, or argininate anion,

more preferably, the acidic amino acid anion comprises at least one of cysteinate anion, methioninate anion; or glutamate anion,

more preferably, the amino acid anion is optionally substituted with one or more substituents.

3. The method according to claim 2, wherein the substituent is selected from at least one of amino, hydroxy, alkyl, alkenyl, phenyl, cyano group, thiocyano group, sulfonyl, or carbonyl;

preferred, the alkyl is a C1-C4 straight chain alkyl, the alkenyl is a C1-C4 straight chain alkenyl, the cyano group is a C1-C5 straight chain cyano group, the thiocyano group is a C1-C4 straight chain thiocyano group, the sulfonyl is a C1-C4 straight chain sulfonyl group, and the carbonyl is a C1-C4 straight chain carbonyl group.

4. The method according to any one of claims 1-3, wherein the ionic liquid is selected from at least one of 1-ethyl-3-methylimidazolium amino acid ionic liquid, cholinium amino acid ionic liquid, tert-butylammonium amino acid ionic liquid, 1-ethyl-1-methylpiperidinium phosphate amino acid ionic liquid, 1-ethyl-1-methylpyrrolidinium amino acid ionic liquid, 1-hydroxyethyl-3-methylimidazolium amino acid ionic liquid, betainium amino acid ionic liquid, and 1-butyl-2,3-dimethylimidazolium amino acid ionic liquid,

preferably, the ionic liquid is selected from at least one of betainium alaninate ionic liquid, cholinium prolinate ionic liquid, cholinium serinate ionic liquid, or cholinium argininate ionic liquid.

5. The method according to any one of claims 1-4, wherein the method further comprises:

concentrating and drying the raffinate of the fractional extraction to give deoxycholic; and/or
back-extracting the extract of the fractional extraction and concentrating and drying the back-extracted phase to give cholic acid.

6. The method according to any one of claims 1-5, wherein in the fractional extraction, the flow ratio of extractant, scrubbing solvent and feed solution is 1: (0.5-5): (0.1-10), preferably 1: (0.75-1): (0.25-5).

7. The method according to any one of claims 1-6, wherein the mass fraction of ionic liquid in the extractant is 0.01%-100%, preferably 0.01%-20%, further preferably 0.05%-5%.

8. The method according to any one of claims 1-7, wherein the total concentration of the cholic acid and deoxycholic acid in the feed solution is 0.1 g/L-100.0 g/L.

9. The method according to any one of claims 1-8, wherein the feed solution further comprises a feed solvent, preferably, the feed solvent is selected from C4-C8 straight or branched chain alcohol compounds, more preferably, the feed solvent is selected from at least one of n-butanol, 2-butanol, iso-butanol, n-pentanol, n-hexanol, 2-hexanol, n-heptanol, iso-octanol, and iso-amyl alcohol; and/or

the extractant further comprises a diluent, preferably the diluent is selected from a polar solvent or a non-polar solvent, more preferably the polar solvent is selected from water and/or dimethyl sulfoxide and the non-polar solvent is selected from n-hexane; and/or
the scrubbing solvent is selected from C4-C8 straight or branched chain alcohol compounds, preferably, selected from at least one of n-butanol, 2-butanol, iso-butanol, n-pentanol, n-hexanol, 2-hexanol, n-heptanol, iso-octanol, and isoamyl alcohol, preferably the scrubbing solvent is the same as the feed solvent.

10. The method according to any one of claims 1-9, wherein the operation temperature of the fractional extraction is 20°C-70°C.

## INTERNATIONAL SEARCH REPORT

| International application No. |
| --- |
| **PCT/CN2024/088970** |

| A. | CLASSIFICATION OF SUBJECT MATTER |
| --- | --- |

C07J 9/00(2006.01)i; C07J 75/00(2006.01)i; B01D 11/04(2006.01)i; B01D 3/40(2006.01)i

According to International Patent Classification (IPC) or to both national classification and IPC

| B. | FIELDS SEARCHED |
| --- | --- |

Minimum documentation searched (classification system followed by classification symbols)

IPC: C07J B01D

Documentation searched other than minimum documentation to the extent that such documents are included in the fields searched

Electronic data base consulted during the international search (name of data base and, where practicable, search terms used)

CNTXT: ENTXTC; ENTXT; CJFD; DWPI; CNABS; CNKI; ISI-Web of Science; 超星读秀, CHAOXING DUXIU; 万方, WANFANG: 浙江大学, 浙江大学衢州研究院, 鲍宗必, 丁泽相, 曹义风, 陈俐吭, 杨启炜, 刘宝鉴, 张治国, 任其龙, 胆酸, 胆烷酸, 去氧胆酸, 离子液体, 氨基酸, 氨酸, 萃取, 分离, cholic acid, deoxycholic acid, CA, DCA, bile acid, ionic liquid?, amino acid, extraction, separation

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | CN 117327137 A (ZHEJIANG UNIVERSITY et al.) 02 January 2024 (2024-01-02)<br>claims 1-10 | 1-10 |
| X | CN 116284193 A (ZHEJIANG UNIVERSITY et al.) 23 June 2023 (2023-06-23)<br>claims 2-10, and description, embodiment 9 | 1-10 |
| A | CN 102718826 A (ZHEJIANG UNIVERSITY) 10 October 2012 (2012-10-10)<br>entire document | 1-10 |
| A | CN 116375784 A (ZHEJIANG UNIVERSITY et al.) 04 July 2023 (2023-07-04)<br>entire document | 1-10 |
| PX | DING, Zexiang. "A Synergistic 'push and pull' Ionic Liquid Biphasic System for Enhanced Extraction Separation of Cholic Acid and Deoxycholic Acid"<br>*Green Chemistry*. Vol. 25, No. 24, 23 November 2023 (2023-11-23), 10472-10484<br>ISSN: 1463-9262,<br>experiment section | 1-10 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **17 July 2024** | **24 July 2024** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2024/088970**

| Patent document cited in search report | | | Publication date (day/month/year) | Patent family member(s) | | | Publication date (day/month/year) |
|---|---|---|---|---|---|---|---|
| CN | 117327137 | A | 02 January 2024 | None | | | |
| CN | 116284193 | A | 23 June 2023 | None | | | |
| CN | 102718826 | A | 10 October 2012 | CN | 102718826 | B | 06 August 2014 |
| CN | 116375784 | A | 04 July 2023 | None | | | |

Form PCT/ISA/210 (patent family annex) (July 2022)